# EUROPÄISCHE PATENTSCHRIFT

(11) **EP 0 539 765 B1**
(45) Veröffentlichungstag und Bekanntmachung des Hinweises auf die Patenterteilung: **20.07.1994**
(21) Anmeldenummer: 92117010.6
(22) Anmeldetag: 06.10.1992
(51) Int. Cl.: C07C 25/13

(54) **Verfahren zur Herstellung von 2,4-Dichlorfluorbenzol**
Process for the preparation of 2,4-dichlorofluorobenzene
Procédé de préparation 2,4-dichloro-fluorobenzène

(30) Priorität: 11.10.1991 DE 4133689
(43) Veröffentlichungstag der Anmeldung: 05.05.1993
(73) Patentinhaber: HOECHST AKTIENGESELLSCHAFT, 65926 Frankfurt am Main (DE)
(72) Erfinder: Papenfuhs, Theodor, Dr., W-6000 Frankfurt/Main (DE); Folz, Georg, Dr., W-6000 Frankfurt/Main (DE); Pfirmann, Ralf, Dr., W-6103 Griesheim (DE)

(56) Entgegenhaltungen:
- EP-A- 0 307 481
- EP-A- 0 355 719

## Beschreibung

Die vorliegende Erfindung betrifft ein Verfahren zur Herstellung von 2,4-Dichlorfluorbenzol in hoher Ausbeute und Reinheit in einem dreistufigen Verfahren, wobei nur das Endprodukt durch Fraktionierung oder Schmelzkristallisation gereinigt wird, die Zwischenstufen aber jeweils in roher Form weiterverarbeitet werden. Hierbei wird die zum Teil aufwendige Isomerentrennung der Zwischenstufen als überflüssig umgangen, weil die auftretenden Isomeren in dasselbe Endprodukt überführt werden. Das gewünschte Endprodukt enthält vor der Fraktionierung lediglich 2 % 2,6-Dichlorfluorbenzol als Verunreinigung.

Das erfindungsgemäß wirtschaftlich herstellbare 2,4-Dichlorfluorbenzol ist ein wertvolles Zwischenprodukt für die Herstellung von antibakteriellen Mitteln der Chinoloncarbonsäurereihe. Es kann durch Acylierung und anschließende Oxidation (DE 3435392; DE 3925036, EP 411252) in 2,4-Dichlor-5-fluorbenzoesäure, die durch literaturbekannte Verfahren in Chinoloncarbonsäurederivate umgewandelt werden kann, überführt werden (DE 3702393; DE 3615767; DE 3601567; DE 3600891; DE 3522406; DE 3517535; DE 3641312). Das durch Acylierung erhältliche 2,4-Dichlor-5-fluoracetophenon kann auch direkt als Basis zur Synthese von antibakteriell wirksamen Verbindungen dienen (EP 131839). Ebenfalls möglich ist die Bromierung des 2,4-Dichlorfluorbenzols sowie anschließender Brom-Cyan-Austausch und Fluorierung zum 2-Chlor-4,5-difluorbenzonitril, das in 2-Chlor-4,5-difluorbenzoesäure umgewandelt wird (EP 433124) und ein günstigeres Vorprodukt für Chinoloncarbonsäurederivate darstellt (EP 342849; EP 321191; EP 303291). Auf diesem Wege kann ebenfalls 2,4,5-Trifluorbenzoesäure hergestellt werden, die in gleicher Weise in antibakteriell wirksame Verbindungen umgewandelt werden kann (J.P. Sanchez, J.M. Domagala, S.E. Hagen, C.L. Heifetz, M.P. Hutt, J.B. Nichols, A.K. Trehan, J. Med. Chem. 31 (1988), 983-991; EP 227088; DE 3600891; DE 3420743; JP 60072885; EP 191185). Möglich ist ebenfalls die Acylierung von 2,4-Dichlorfluorbenzol mittels Phosgen zum 2,4-Dichlor-5-fluorbenzoylchlorid (JP 01226851), das dann entweder durch indirekte Hydrolyse in 2,4-Dichlor-5-fluorbenzoesäure (JP 01226851, loc. cit.) oder durch Chlor-Fluor-Austausch-Reaktion und alkalische Hydrolyse in 2,4,5-Trifluorbenzoesäure umgewandelt werden kann (DE 34 20 796).

Bisher wurde 2,4-Dichlorfluorbenzol durch Sandmeyer-Reaktion aus 3-Chlor-4-fluoranilin hergestellt (Houben-Weyl-Müller, Methoden der Organischen Chemie Bd. 5/3 (1963), 846-853). Sandmeyer-Reaktionen gehen aber stets mit geringen Raum-Zeit-Ausbeuten und hohen Abwasserbelastungen einher. 3-Chlor-4-fluoranilin wurde durch Reduktion aus 3-Chlor-4-fluornitrobenzol hergestellt, das als nicht zu reinigendes Zwischenprodukt bei dem erfindungsgemäßen Verfahren auftritt und entweder durch Chlorierung von isomerenreinem 4-Fluornitrobenzol (EP 307481, loc. cit.; van de Lande, Rec. Trav. Chim. Pays-Bas, 51 (1932), 98-101; Rinkes, Chemisches Zentralblatt (1914/II), 1432) oder durch Halex-(Chlor, Fluor)-Reaktion aus 3,4-Dichlornitrobenzol (DE 2938939; US 4229365), das leicht durch Nitrierung von o-Dichlorbenzol erhalten wird und in großen Mengen schon technisch verfügbar ist, hergestellt werden kann.

EP-A-355 719 beschreibt die reduktive Chlorierung von fluorhaltigen Nitrobenzolen. Insbesondere wird in Beispiel 3 die Herstellung von 2,4-Dichlorofluorbenzol ausgehend von 3,4-Dichlornitrobenzol durch Halex-(Chlor, Fluor)-Reaktion und reduktive Chlorierung offenbart.

Es wurde nun gefunden, daß man 2,4-Dichlorfluorbenzol in hohen Ausbeuten und hoher Reinheit in einem Dreistufenverfahren ohne intermediäre Trennung der gebildeten Isomeren herstellen kann, indem man (1) 1 Mol Fluorbenzol mit einer Mischung, bestehend aus 35 bis 65 Gewichtsteilen 50 bis 90%iger, vorzugsweise 65 bis 75%iger Schwefelsäure, 35 bis 65 Gewichtsteilen Nitriersäure, bestehend aus 35 bis 55 Gewichtsteilen 95 bis 98%iger Schwefelsäure und 45 bis 65 Gewichtsteilen 96 bis 98%iger Salpetersäure, mit der Maßgabe, daß 0,8 bis 2,0 Äquivalente, vorzugsweise 0,9 bis 1,2 Äquivalente nitrierendes Agens NO₂⁺ pro Mol Fluorbenzol zur Anwendung gelangen, bei Temperaturen von 20 bis 90°C, vorzugsweise von 50 bis 70°C, gegebenenfalls in Gegenwart eines Lösungs- oder Verdünnungsmittels, zum Nitrofluorbenzol nitriert, (2) auf je 100 g des so erhaltenen Nitrofluorbenzol-Rohgemisches 25 g bis 150 g, vorzugsweise 27 g bis 50 g besonders bevorzugt 27 bis 33 g Chlor oder äquivalente Mengen eines chlorabgebenden Mittels in Gegenwart eines Kernchlorierungskatalysators, gegebenenfalls in Anwesenheit eines Verdünnungsmittels, bei Temperaturen von 20 bis 100°C, vorzugsweise von etwa 50 bis 70°C einwirken läßt, und (3) auf je 100 g des erhaltenen Chlorfluornitrobenzol-Rohgemisches nach Entfernung des Kernchlorierungskatalysators 18 g bis 203 g, vorzugsweise 22 g bis 81,2 g, besonders bevorzugt 22 g bis 40,6 g Chlor oder äquivalente Mengen eines chlorabgebenden Mittels, gegebenenfalls in Anwesenheit eines Verdünnungsmittels und gegebenenfalls in Gegenwart eines wasserentziehenden Mittels und/oder eines fluoridabfangenden Mittels, bei Temperaturen von 110°C bis 220°C, vorzugsweise von 175° bis 190°C, einwirken läßt (denitrierende Chlorierung) und anschließend das 2,4-Dichlorfluorbenzol durch fraktionierte Destillation oder Schmelzkristallisation isoliert.

Nachstehend seien nähere Einzelheiten zu den einzelnen Verfahrensstufen angegeben.
Zur ersten Stufe (Nitrierung des Fluorbenzols): Es ist zweckmäßig, das Fluorbenzol in einer Emulsion, bestehend aus der genannten Schwefelsäure und Nitriersäure sowie dem gegebenenfalls anwesenden Lösungs- oder Verdünnungsmittel zu nitrieren. Als Lösungs- oder Verdünnungsmittel können beispielsweise Nitrobenzol, chlorierte Nitrobenzole oder fluorierte Nitrobenzole, beispielsweise ein Nitrofluorbenzolrohgemisch, wie es z.B. beim erfindungsgemäßen Verfahren entsteht, Dichlormethan, Trichlormethan, Tetrachlormethan, 1,2-Dichlorethan und niedere Paraffine, wie z.B. n-Hexan, Oktan oder ähnliches zur Anwendung gelangen. Die Nitrierung kann sowohl diskontinuierlich (batchweise) als auch kontinuierlich durchgeführt werden. Bei der kontinuierlichen Arbeitsweise wird die Nitriersäure vorzugsweise in solchen Mengen eingesetzt, daß 1,0 bis 1,1 Äquivalente nitrierendes Agens (NO₂⁺) pro Mol Fluorbenzol zur Anwendung gelangen.

Außerdem ist es zweckmäßig in einer Kaskade, bestehend aus vorzugsweise 3 bis 5 Kesseln, zu arbeiten.

Bei der diskontinuierlichen (batchweisen) Nitrierung wird nichtumgesetztes Fluorbenzol entweder durch höhere Überschüsse an Nitriersäure abgefangen oder nach Destillation von umgesetztem Material abgetrennt und zurückgeführt. Die Gesamtausbeuten bei der ersten Stufe bei diskontinuierlicher Arbeitsweise betragen mehr als 95 %, bei kontinuierlicher Arbeitsweise über 99 %, bezogen auf eingesetztes Fluorbenzol.

Im Gegensatz zu dem in EP 307 481 beschriebenen Verfahren werden die hierbei angefallenen Isomeren nicht getrennt, was wegen der geringen Siedepunktdifferenz einen erheblichen Fraktionaufwand erfordern würde, sondern werden im Gemisch der zweiten Stufe (Chlorierung mittels Chlor oder einem chlorabgebenden Mittel) unterworfen. Als chlorabgebende Mittel kommen beispielsweise Antimonpentachlorid, Jodtrichlorid, Schwefeldichlorid, Dischwefeldichlorid oder Mangantetrachlorid oder Mischungen daraus in Frage.

Die Chlorierung (in zweiter Stufe des erfindungsgemäßen Verfahrens) wird in Gegenwart eines Kernchlorierungskatalysators, wie Eisen, Eisen(II)-chlorid, Eisen(III)-chlorid, Jod, Jodtrichlorid, Jodpentachlorid, Antimontrichlorid, Antimonpentachlorid oder Aluminium(III)-chlorid oder eines Gemisches daraus, vorzugsweise in Gegenwart von Eisen(III)-chlorid, durchgeführt. Die Chlorierung der zweiten Reaktionsstufe wird bevorzugt in Abwesenheit eines Verdünnungsmittels durchgeführt. Sie kann aber auch in Anwesenheit eines inerten organischen Lösungsmittels, wie beispielsweise Tetrachlormethan, Chloroform, 1,2-Dichlorethan, Oleum, Schwefelsäure oder Chlorsulfonsäure, vorgenommen werden. Das in zweiter Stufe erhaltene Produktgemisch besteht zu etwa 87 % aus 3-Chlor-4-fluornitrobenzol, zu etwa 11 % aus 2-Fluor-5-chlornitrobenzol und zu etwa 2 % aus 2-Fluor-3-chlornitrobenzol. Weitere Komponenten, wie bei der Chlorierung der zweiten Stufe nicht weiter umgesetztes 2,4-Dinitrofluorbenzol, sind in weniger als 0,1 % der Gesamtmenge enthalten. Auch auf dieser Stufe (die Gesamtausbeuten an Mischung betragen über 90 %) betragen die Ausbeuten über 95 %. Eine Trennung der erhaltenen Verbindungen erfolgt nicht. Die Entfernung des Kernchlorierungskatalysators erfolgt - nach vorheriger Abtrennung des gegebenenfalls anwesenden Verdünnungsmittels durch Phasentrennung und Wäsche oder durch Abdestillieren - durch mehrmaliges Auswaschen der organischen Phase mit Wasser und/oder verdünnter Natronlauge. Es bietet sich auch an, die in zweiter Stufe anfallende Produktmischung durch einfache Destillation vom im Sumpf zurückbleibenden Katalysator abzutrennen; der Siedebereich der Produktmischung liegt bei 75 bis 100°C bei 4 mbar.

Arbeitet man hierbei mit reinen Verbindungen, so ist die denitrierende Chlorierung durch einige Beispiele in der Literatur auch für 2,4-Dichlorfluorbenzol als Produkt bekannt (N.N. Voroshtsov, G.G. Yakobson, N.I. Krizhechkovskaya, A. I. D'yachenko, I. V. Shikanova, Zh. Obshch. Khim. 31(4), 1222-1226 (1961), CA 55 (1961), 24605i; N. N. Voroshtsov, G. G. Yakobson, N. I. Krizhechkovskaya, Khim. Nauka i Prom. 3 (1958), 404-405, CA 52 (1958), 19987h; V. A. Solenko, N. N. Voroshtsov, G. G. Yakobson, Izv. Sibirsk. Otd. Akad. Nauk SSSR (1962/10), 87-90, CA 59, 1507b; L. C. Kun, K. G. Lo, P. G. Pil, C. J. Pung, Hwahak Kwa Hwahak Kongop (1972/5), 251-253, CA 79 (1973), 31599n; EP 163230; EP 355719; EP 180057; EP 150587). Es war jedoch überraschend, daß das in den zwei vorhergehenden Stufen nicht gereinigte Rohgemisch ebenso glatt die Reaktion einging und sich kontinuierlich umsetzen ließ, was bei Anwesenheit von Verunreinigungen nicht immer gelingt.

Der Ersatz der Nitrogruppen durch Chlor in der dritten Reaktionsstufe erfolgt bei Temperaturen von 110 bis 200°C. Bevorzugt wird bei einer Sumpftemperatur von 175 bis 190°C gearbeitet, weil hierbei 2,4-Dichlorfluorbenzol als Rohprodukt gleichmäßig abdestilliert werden kann. Die bevorzugt eingesetzten Chlormengen betragen bei der kontinuierlichen Arbeitsweise von 50 bis 200 ml/g·h. Diese letzte Stufe kann aber auch diskontinuierlich (batchweise) durch Einchlorierung im Unterschuß (20 bis 30 %) unter späterem Abdestillieren des zuerst siedenden Produktes erfolgen. Es ist zweckmäßig, dem Chlorierungssumpf zur Verhinderung der Fluorwasserstoffkorrosion, die bei Verwendung der erfindungsgemäßen Rohgemische gegenüber den reinen Verbindungen verstärkt werden kann, wasserentziehende und/oder fluoridabfangende Mittel zuzusetzen. Calciumsalze, wie Calciumchlorid, Calciumsulfat oder Calciumhydroxid, sowie Siliciumdioxid werden bevorzugt als fluoridabfangende Mittel verwendet. Als wasserentziehende Mittel können Phosphorpentoxid oder Phosphorpentachlorid eingesetzt werden.

Die Gesamtausbeuten an 2,4-Dichlorfluorbenzol, bezogen auf eingesetztes Fluorbenzol, betragen 85 bis 90 %; der Reingehalt des abdestillierten Rohproduktes ist größer als 97 %. Bei der Fraktionierung läßt sich das in Anteilen von etwa 2 % mitentstandene 2,6-Dichlorfluorbenzol (Sdp. 179°C) ebenso wie weitere Minderkomponenten (siehe Verfahrensbeschreibung) als nach dem gewünschten Produkt (Sdp. 171°C) siedende Komponente abtrennen (Normaldruck). Man kann die Fraktionierung aber auch im Vakuum durchführen. Die dabei sich verringernde Siedepunktsdifferenz muß durch Verwendung einer Kolonne mit höherer Anzahl an Trennstufen ausgeglichen werden. Ebenfalls möglich ist die Trennung der Isomeren durch Schmelzkristallisation, da die Hauptverunreinigung mit 41°C einen wesentlich höheren Erstarrungspunkt als das gewünschte Produkt aufweist (unter -25°C).

Das nachstehende Beispiel dient zur Erläuterung des Verfahrens, ohne es darauf zu beschränken.

### Beispiel

### a) Nitrierung von Fluorbenzol

In einer Kaskade aus 3 Rührapparaten wird Fluorbenzol (288 g/h, 3 Mol) in Emulsion mit Salpetersäure (96 %, 204 g/h) und Schwefelsäure (98 %, 175 g/h) bei Temperaturen von 45 bis 50°C (1. Kessel), 50 bis 55°C (2. Kessel) und 55 bis 60°C (3. Kessel) nitriert. Die mittlere Verweilzeit wird auf etwa 3 h eingestellt. Die Emulsion wird aufrecht erhalten durch Zudosierung von 392 g/h 72proz. Schwefelsäure. Pro Stunde können 1057 g Nitriergemisch entnommen werden, das neben etwa 625 g 72proz. Schwefelsäure und etwa 9 g Salpetersäure 423 g Nitrofluorbenzol-Isomerengemisch enthält. Die obere organische Phase wird mit 250 g Wasser, 250 g 2,5proz. Natriumhydrogencarbonatlösung und wieder 250 g Wasser neutral gewaschen, die untere Phase der Abfallsäure kann im Kreis geführt werden. Es verbleiben 420 g (318 ml) organische Phase (Nitrofluorbenzol, 99,2 % bezogen auf Fluorbenzol) die 0,3 % m-Nitrofluorbenzol, 85,7 % p-Nitrofluorbenzol und 0,4 % 2,4-Dinitrofluorbenzol enthält. Die Mischung wird in dieser Form in die nachfolgende Reaktion eingesetzt.
Nitriert man batchweise bei Temperaturen zwischen etwa 20 und etwa 80°C, vorzugsweise zwischen etwa 50 und etwa 70°C, so gelangt man zum selben Ergebnis, wenn man den Überschuß an Nitriersäure so wählt, daß kein Fluorbenzol verbleibt oder bei geringerem Überschuß das nicht umgesetzte Fluorbenzol durch einfache Destillation aus dem Nitriergemisch entfernt und zurückführt.

### b) Chlorierung des Nitrofluorbenzol-Rohgemisches

Die aus der Stufe a) erhaltene Menge an Nitrofluorbenzolrohgemisch (420 g) wird mit 3 g Eisenspänen versetzt. Anschließend wird bei 60°C solange Chlor eingeleitet, bis sich durch GC kein Nitrofluorbenzol mehr nachweisen läßt (im allgemeinen nach 9 bis 11 h). Durch Waschen der organischen Phase mit Wasser (250 g) und 5prozentiger Natronlauge (250 g) wird der Katalysator entfernt. Es verbleiben 518 g organisches Gemisch aus Chlorfluornitrobenzolen, das ohne weitere Behandlung in die Stufe c) eingesetzt wird. Das Gemisch enthält 85 bis 87 % 3-Chlor-4-fluornitrobenzol, 10 bis 12 % 2-Fluor-5-chlornitrobenzol, 1 bis 3 % 2-Fluor-3-chlornitrobenzol, weniger als 0,1 % 2,4-Dinitrofluorbenzol und weniger als 0,3 % Chlorierungsprodukte des 3-Fluornitrobenzols, wie 2-Chlor-5-fluornitrobenzol, 3-Chlor-5-fluornitrobenzol, 3-Fluor-4-chlornitrobenzol und 2-Chlor-3-fluornitrobenzol; dichlorierte Anteile sind ebenfalls unter 0,3 % zu veranschlagen.

Verwendet man anstatt Eisenspänen 5,5 g Eisen(III)chlorid oder 3 g Antimon(V)chlorid oder 1 g Jodtrichlorid oder 6 g Aluminiumtrichlorid oder ein Gemisch dieser Katalysatoren in entsprechenden molaren Mengen, so gelangt man im wesentlichen zum selben Ergebnis. Chloriert man bei 50°C (70°C), so läßt sich nach 15 (7 bis 8) Stunden keine Ausgangsverbindung mehr nachweisen. Die Isomerenverhältnisse hängen in diesem Bereich kaum von der Temperatur ab, die Ausbeuten gehen bei erhöhter Temperatur um 3 % (70°C) bzw. 6 % (90°C) zurück. Zur mit Sicherheit vollständigen Abtrennung von Katalysatorresten kann die Rohproduktmischung im Vakuum überdestilliert werden (Siedebereich 75 bis 100°C bei 4 mbar entsprechend 400 Pa).

### c) Denitrierende Chlorierung

Die aus der Verfahrensstufe b) erhaltene Menge (518 g) Rohgemisch wird auf 180°C erhitzt. In das Gemisch wird ein Chlorgasstrom von 7 l/h eingeleitet. Nach etwa 2 h hat sich genügend Produkt gebildet, das über eine 40 cm lange mit Glaswendeln gefüllte Kolonne abdestilliert wird (Siedebereich 165 bis 175°C). Es wird nun in gleichem Maße, wie Produktmischung abgenommen wird, aus weiteren Kernchlorieransätzen (Verfahrensstufe b) erhaltenes Material nachdosiert (45 g/h), um die kontinuierliche Fahrweise zu gewährleisten. So können etwa 43 g/h Produktmischung gewonnen werden, mit dieser gehen eine stark saure wäßrige Phase und nitrose Gase über. Nach stündlicher Abtrennung dieser Phase wird die organische Phase mit Wasser (zweimal 50 g) und 10proz. Natronlauge (50 g) neutral gewaschen. Die organische Phase, die zu etwa 97 bis etwa 98 % aus 2,4-Dichlorfluorbenzol, zu etwa 2 % aus 2,6-Dichlorfluorbenzol und zu weniger als 0,2 % aus weiteren Minderkomponenten, wie 3,4- und 3,5-Dichlorfluorbenzol besteht, wird anschließend über eine 30-bödige Kolonne bei Normaldruck fraktioniert. Zuerst geht bei 165 bis 170°C (Sumpftemperatur 180 bis 185°C) ein Vorlauf über. Bei 171 bis 172°C gewinnt man 2,4-Dichlorfluorbenzol mit einem Reingehalt von über 99,5 %; höhere Reingehalte lassen sich durch Abnahme eines größeren Vorlaufes bzw. Fraktionierung bei höherem Rücklaufverhältnis erreichen. Nach dem gewünschten Produkt geht bei 179°C 2,6-Dichlorfluorbenzol über, die erhaltenen Mischfraktionen werden dabei zurückgeführt. Die Ausbeute an 2,4-Dichlorfluorbenzol beträgt 89 % für die Stufe der denitrierenden Chlorierung, bezogen auf Rohgemisch, und 86,5 %, bezogen auf in den ersten Verfahrensschritt eingesetztes Fluorbenzol. Im Reaktionssumpf reichert sich ein TrichlorfluorbenzolIsomerengemisch an, das nach etwa 200 h kontinuierlicher Betriebszeit ausgeschleust wird. Dies gelingt durch Fraktionierung des Sumpfes, wobei der dabei anfallende Destillationssumpf verworfen wird.

Arbeitet man unter Zusatz von 5 g Calciumchlorid oder 8 g Siliciumdioxid, so erhält man dasselbe Ergebnis; der Abtrag in der verwendeten Apparatur durch Korrosion läßt sich aber wesentlich verringern.

Anstatt das gewaschene Rohprodukt zu fraktionieren, kann man durch Schmelzkristallisation bei etwa -15°C die Verunreinigungen fast vollständig abtrennen. Die flüssige Phase ist 2,4-Dichlorfluorbenzol mit einem Reingehalt von mehr als 99,8 %.

## Patentansprüche

1. Verfahren zur Herstellung von 2,4-Dichlorfluorbenzol in hohen Ausbeuten und hoher Reinheit in einem Dreistufenverfahren ohne intermediäre Trennung der gebildeten Isomeren, dadurch gekennzeichnet, daß man (1) 1 Mol Fluorbenzol mit einer Mischung, bestehend aus 35 bis 65 Gewichtsteilen 50 bis 90 %iger Schwefelsäure und 35 bis 65 Gewichtsteilen Nitriersäure, bestehend aus 35 bis 55 Gewichtsteilen 95 bis 98 %iger Schwefelsäure und 45 bis 65 Gewichtsteilen 96 bis 98 %iger Salpetersäure, mit der Maßgabe, daß 0,8 bis 2,0 Äquivalente nitrierendes Agens NO₂⁺ pro Mol Fluorbenzol zur Anwendung gelangen, bei Temperaturen von 20 bis 90 °C, gegebenenfalls in Gegenwart eines Lösungs- oder Verdünnungsmittels, zum Nitrofluorbenzol nitriert, (2) auf je 100 g des erhaltenen Nitrofluorbenzol-Rohgemisches 25 g bis 150 g Chlor oder äquivalente Mengen eines chlorabgebenden Mittels in Gegenwart eines Kernchlorierungskatalysators, gegebenenfalls in Anwesenheit eines Verdünnungsmittels, bei Temperaturen von 20 bis 100°C einwirken läßt, und (3) auf je 100 g des erhaltenen Chlorfluornitrobenzol-Rohgemisches nach Entfernung des Kernchlorierungskatalysators 18 g bis 203 g Chlor oder äquivalente Mengen eines chlorabgebenden Mittels, gegebenenfalls in Anwesenheit eines Verdünnungsmittels und gegebenenfalls in Gegenwart eines wasserentziehenden Mittels und/oder eines fluoridabfangenden Mittels, bei Temperaturen von 110° bis 220°C einwirken läßt (denitrierende Chlorierung) und anschließend das 2,4-Dichlorfluorbenzol durch fraktionierte Destillation oder Schmelzkristallisation isoliert.

2. Verfahren nach Anspruch 1, dadurch gekennzeichnet, daß die zur Nitrierung verwendete Mischung aus Schwefelsäure und Nitriersäure eine 65 bis 75%ige Schwefelsäure enthält.

3. Verfahren nach mindestens einem der Ansprüche 1 und 2, dadurch gekennzeichnet, daß mit der angewandten Nitriersäure 0,9 bis 1,2 Äquivalent nitrierendes Agens NO₂⁺ pro Mol Fluorbenzol zur Anwendung gelangen.

4. Verfahren nach mindestens einem der Ansprüche 1 und 2, dadurch gekennzeichnet, daß man kontinuierlich oder diskontinuierlich nitriert.

5. Verfahren nach mindestens einem der Ansprüche 1, 2 und 4, dadurch gekennzeichnet, daß bei kontinuierlicher Nitrierung mit der angewandten Nitriersäure 1,0 bis 1,1 Äquivalente nitrierendes Agens NO₂⁺ pro Mol Fluorbenzol zur Anwendung gelangen.

6. Verfahren nach mindestens einem der Ansprüche 1 bis 5, dadurch gekennzeichnet, daß man bei Temperaturen von 50 bis 70°C nitriert.

7. Verfahren nach mindestens einem der Ansprüche 1 bis 6, dadurch gekennzeichnet, daß man in Gegenwart von Nitrobenzol, chlorierten Nitrobenzolen, fluorierten Nitrobenzolen, einem Nitrofluorbenzolrohgemisch wie es beim erfindungsgemäßen Verfahren entsteht, Dichlormethan, Trichlormethan, Tetrachlormethan, 1,2-Dichlorethan oder niedere Paraffinen als Lösungs- oder Verdünnungsmittel nitriert.

8. Verfahren nach mindestens einem der Ansprüche 1 bis 7, dadurch gekennzeichnet, daß man bei kontinuierlicher Nitrierung in einer Kaskade, bestehend aus 3 bis 5 Kesseln, arbeitet.

9. Verfahren nach mindestens einem der Ansprüche 1 bis 8, dadurch gekennzeichnet, daß man die Chlorierung des bei der Nitrierung erhaltenen Nitrofluorbenzol-Rohgemisches in Gegenwart von Eisen, Eisen(II)-chlorid, Eisen(III)-chlorid, Jod, Jodtrichlorid, Jodpentachlorid, Antimontrichlorid, Antimonpentachlorid oder Aluminium(III)-chlorid oder Mischungen daraus vornimmt.

10. Verfahren nach mindestens einem der Ansprüche 1 bis 9, dadurch gekennzeichnet, daß man auf je 100 g des bei der Nitrierung erhaltenen Nitrofluorbenzol-Rohgemisches 27 g bis 50 g Chlor oder äquivalente Mengen eines chlorabgebenden Mittels einwirken läßt.

11. Verfahren nach mindestens einem der Ansprüche 1 bis 9, dadurch gekennzeichnet, daß man auf je 100 g des bei der Nitrierung erhaltenen Nitrofluorbenzol-Rohgemisches 27 bis 33 g Chlor oder äquivalente Mengen eines chlorabgebenden Mittels einwirken läßt.

12. Verfahren nach mindestens einem der Ansprüche 1 bis 11, dadurch gekennzeichnet, daß man das Chlor oder das chlorabgebende Mittel auf das bei der Nitrierung erhaltene Nitrofluorbenzol-Rohgemisch bei Temperaturen von 50 bis 70°C einwirken läßt.

13. Verfahren nach mindestens einem der Ansprüche 1 bis 12, dadurch gekennzeichnet, daß man das bei der Nitrierung erhaltene Nitrofluorbenzol-Rohgemisch in Gegenwart von Tetrachlormethan, 1,2-Dichlorethan, Chloroform, Dichlormethan, Chlorsulfonsäure, Oleum oder Schwefelsäure chloriert.

14. Verfahren nach mindestens einem der Ansprüche 1 bis 13, dadurch gekennzeichnet, daß man die denitrierende Chlorierung bei Temperaturen von 175 bis 190°C vornimmt.

15. Verfahren nach mindestens einem der Ansprüche 1 bis 14, dadurch gekennzeichnet, daß man auf je 100 g des in zweiter Stufe erhaltenen Chlorfluornitrobenzol-Rohgemisches 22 g bis 81,2 g Chlor oder äquivalente Mengen eines chlorabgebenden Mittels einwirken läßt.

16. Verfahren nach mindestens einem der Ansprüche 1 bis 15, dadurch gekennzeichnet, daß man auf je 100 g des in zweiter Stufe erhaltenen Chlorfluornitrobenzol-Rohgemisches 22 g bis 40,6 g Chlor oder äquivalente Mengen eines chlorabgebenden Mittels einwirken läßt.

17. Verfahren nach mindestens einem der Ansprüche 1 bis 16, dadurch gekennzeichnet, daß man die denitrierende Chlorierung in Gegenwart von Phosphorpentoxid oder Phosphorpentachlorid als wasserentziehendem Mittel durchführt.

18. Verfahren nach mindestens einem der Ansprüche 1 bis 17, dadurch gekennzeichnet, daß man die denitrierende Chlorierung in Gegenwart von Calciumchlorid, Calciumhydroxid, Calciumsulfat oder Siliciumdioxid als fluoridabfangendem Mittel durchführt.

19. Verfahren nach mindestens einem der Ansprüche 1 bis 18, dadurch gekennzeichnet, daß man die denitrierende Chlorierung kontinuierlich oder diskontinuierlich durchführt.

## Claims

1. A process for the preparation of 2,4-dichlorofluorobenzene in high yields and high purity in a three-stage process without intermediate separation of the isomers formed, wherein (1) 1 mol of fluorobenzene is nitrated to give nitrofluorobenzene using a mixture comprising 35 to 65 parts by weight of 50 to 90% strength sulfuric acid and 35 to 65 parts by weight of nitrating acid comprising 35 to 55 parts by weight of 95 to 98% strength sulfuric acid and 45 to 65 parts by weight of 96 to 98% strength nitric acid, with the proviso that 0.8 to 2.0 equivalents of nitrating agent NO₂⁺ are used per mole of fluorobenzene, at temperatures of 20 to 90°C, optionally in the presence of a solvent or diluent, (2) 25 g to 150 g of chlorine, or equivalent amounts of a chlorine releasing agent, are allowed to act on each 100 g of the crude nitrofluorobenzene mixture obtained, in the presence of a ring chlorinating catalyst, optionally in the presence of a diluent, at temperatures of 20 to 100°C, and (3) 18 g to 203 g of chlorine or equivalent amounts of a chlorine releasing agent are allowed to act on each 100 g of the crude chlorofluoronitrobenzene mixture obtained (denitrating chlorination), after removal of the ring chlorinating catalyst, optionally in the presence of a diluent and optionally in the presence of a dehydrating agent and/or a fluoride scavenger, at temperatures of 110°C to 220°C and then the 2,4-dichlorofluorobenzene is isolated by fractional distillation or melt crystallization.

2. The process as claimed in claim 1, wherein the mixture used for nitrating comprising sulfuric acid and nitrating acid contains an 65 to 75% strength sulfuric acid.

3. The process as claimed in at least one of claims 1 and 2, wherein 0.9 to 1.2 equivalents of the nitrating agent NO₂⁺ per mole of fluorobenzene are used with the nitrating acid used.

4. The process as claimed in at least one of claims 1 and 2, wherein nitration is carried out continuously or batchwise.

5. The process as claimed in at least one of claims 1, 2 and 4, wherein in the case of continuous nitration, 1.0 to 1.1 equivalents of the nitrating agent NO₂⁺ per mole of fluorobenzene are used with the nitrating acid used.

6. The process as claimed in at least one of claims 1 to 5, wherein nitration is carried out at temperatures of 50 to 70°C.

7. The process as claimed in at least one of claims 1 to 6, wherein nitration is carried out in the presence of nitrobenzene, chlorinated nitrobenzenes, fluorinated nitrobenzenes, a crude nitrofluorobenzene mixture such as is produced in the process according to the invention, dichloromethane, trichloromethane, tetrachloromethane, 1,2-dichloroethane or lower paraffins as solvent or diluent.

8. The process as claimed in at least one of claims 1 to 7, which in the case of continuous nitration is carried out in a cascade comprising 3 to 5 tanks.

9. The process as claimed in at least one of claims 1 to 8, wherein chlorination of the crude nitrofluorobenzene mixture obtained on nitration is performed in the presence of iron, iron(II) chloride, iron(III) chloride, iodine, iodine trichloride, iodine pentachloride, antimony trichloride, antimony pentachloride or aluminum(III) chloride or mixtures thereof.

10. The process as claimed in at least one of claims 1 to 9, wherein 27 g to 50 g of chlorine or equivalent amounts of a chlorine releasing agent are allowed to act on each 100 g of the crude nitrofluorobenzene mixture obtained on nitration.

11. The process as claimed in at least one of claims 1 to 9, wherein 27 to 33 g of chlorine or equivalent amounts of a chlorine releasing agent are allowed to act on each 100 g of the crude nitrofluorobenzene mixture obtained on nitration.

12. The process as claimed in at least one of claims 1 to 11, wherein the chlorine or chlorine releasing agent is allowed to act on the crude nitrofluorobenzene mixture, obtained on nitration, at temperatures of 50 to 70°C.

13. The process as claimed in at least one of claims 1 to 12, wherein the crude nitrofluorobenzene mixture obtained on nitration is chlorinated in the presence of tetrachloromethane, 1,2-dichloroethane, chloroform, dichloromethane, chlorosulfonic acid, oleum or sulfuric acid.

14. The process as claimed in at least one of claims 1 to 13, wherein the denitrating chlorination is carried out at temperatures of 175 to 190°C.

15. The process as claimed in at least one of claims 1 to 14, wherein 22 g to 81.2 g of chlorine or equivalent amounts of a chlorine releasing agent are allowed to act on each 100 g of the crude chlorofluoronitrobenzene mixture obtained in the second stage.

16. The process as claimed in at least one of claims 1 to 15, wherein 22 g to 40.6 g of chlorine or equivalent amounts of a chlorine releasing agent are allowed to act on each 100 g of the crude chlorofluoronitrobenzene mixture obtained in the second stage.

17. The process as claimed in at least one of claims 1 to 16, wherein the denitrating chlorination is performed in the presence of phosphorus pentoxide or phosphorus pentachloride as dehydrating agent.

18. The process as claimed in at least one of claims 1 to 17, wherein the denitrating chlorination is performed in the presence of calcium chloride, calcium hydroxide, calcium sulfate or silicon dioxide as fluoride scavenger.

19. The process as claimed in at least one of claims 1 to 18, wherein the denitrating chlorination is performed continuously or batchwise.

## Revendications

1. Procédé de préparation du 2,4-dichlorofluorobenzène avec des rendements et une pureté élevés dans un procédé en trois étapes sans séparation intermédiaire des isomères formés, caractérisé en ce que (1) on nitre une mole de fluorobenzène avec un mélange constitué de 35 à 65 parties en poids d'acide sulfurique à 50 à 90 % et de 35 à 65 parties en poids d'un acide de nitration constitué de 35 à 55 parties en poids d'acide sulfurique à 95 à 98 % et de 45 à 65 parties en poids d'acide nitrique à 96 à 98 %, sous réserve d'utiliser 0,8 à 2,0 équivalents de l'agent nitrant NO₂* par mole de fluorobenzène, à des températures de 20 à 90 °C, le cas échéant en présence d'un agent de dissolution ou de dilution, pour donner le nitrofluorobenzène, (2) sur 100 g à chaque fois du mélange brut de nitrofluorobenzène obtenu on fait réagir 25 à 150 g ou des quantités équivalentes d'un agent cédant du chlore, en présence d'un catalyseur de chloration dans le noyau, le cas échéant en présence d'un diluant, à des températures de 20 à 100 °C, et (3) sur 100 g à chaque fois du mélange brut de chlorofluoronitrobenzènes obtenu après élimination du catalyseur de chloration dans le noyau, on fait réagir 18 g à 203 g de chlore ou des quantités équivalentes d'un agent cédant du chlore, le cas échéant en présence d'un diluant et le cas échéant en présence d'un agent éliminant l'eau et/ou d'un agent fixant le fluorure, à des températures de 110 °C à 220 °C (chloration dénitrante) puis on isole le 2,4-dichlorofluorobenzène par distillation fractionnée ou cristallisation à l'état fondu.

2. Procédé selon la revendication 1, caractérisé en ce que le mélange d'acide sulfurique et d'acide sulfonitrique utilisé pour la nitration contient un acide sulfurique à 65 à 75 %.

3. Procédé selon au moins une des revendications 1 et 2, caractérisé en ce qu'on utilise avec le mélange de nitration utilisé 0,9 à 1,2 équivalent d'un agent nitrant NO₂⁺ par mole de fluorobenzène.

4. Procédé selon au moins une des revendications 1 et 2, caractérisé en ce qu'on nitre en continu ou en discontinu.

5. Procédé selon au moins une des revendications 1, 2 et 4, caractérisé en ce que dans le cas de la nitration continue avec le mélange de nitration utilisé, on utilise 1,0 à 1,1 équivalent d'agent nitrant NO₂⁺ par mole de fluorobenzène.

6. Procédé selon au moins une des revendications 1 à 5, caractérisé en ce que l'on nitre à des températures de 50 à 70 °C.

7. Procédé selon au moins une des revendications 1 à 6, caractérisé en ce que l'on nitre en présence de nitrobenzène, de nitrobenzènes chlorés, de nitrobenzènes fluorés, d'un mélange brut de nitrofluorobenzènes tel qu'il se forme dans le procédé conforme à l'invention, de dichlorométhane, de trichlorométhane, de tétrachlorométhane, de 1,2-dichloréthane ou de paraffines inférieures comme solvant ou diluant.

8. Procédé selon au moins l'une des revendications 1 à 7, caractérisé en ce que dans le cas de la nitration continue, on opère dans une cascade constituée de 3 à 5 autoclaves.

9. Procédé selon au moins une des revendications 1 à 8, caractérisé en ce qu'on effectue la chloration du mélange brut de nitrofluorobenzène, obtenu lors de la nitration, en présence de fer, de chlorure de fer(II), de chlorure de fer(III), d'iode, de trichlorure d'iode, de pentachlorure d'iode, de trichlorure d'antimoine, de pentachlorure d'antimoine ou de chlorure d'aluminium(III) ou de mélanges de ceux-ci.

10. Procédé selon au moins une des revendications 1 à 9, caractérisé en ce qu'on fait agir, sur 100 g à chaque fois du mélange brut de nitrofluorobenzène obtenu lors de la nitration, 27 g à 50 g de chlore ou des quantités équivalentes d'un agent cédant du chlore.

11. Procédé selon au moins une des revendications 1 à 9, caractérisé en ce qu'on fait agir, sur 100 g à chaque fois du mélange brut de nitrofluorobenzène obtenu lors de la nitration, 27 g à 33 g de chlore ou des quantités équivalentes d'un agent cédant du chlore.

12. Procédé selon au moins une des revendications 1 à 11, caractérisé en ce qu'on fait agir le chlore ou l'agent cédant du chlore sur le mélange brut de nitrofluorobenzène obtenu lors de la nitration à des températures de 50 à 70 °C.

13. Procédé selon au moins une des revendications 1 à 12, caractérisé en ce qu'on chlore le mélange brut de nitrofluorobenzène obtenu en présence de tétrachlorométhane, de l,2-dichloréthane, de chloroforme, de dichlorométhane, d'acide chlorosulfonique, d'oléum ou d'acide sulfurique.

14. Procédé selon au moins une des revendications 1 à 13, caractérisé en ce qu'on effectue la chloration dénitrante à des températures de 175 à 190 °C.

15. Procédé selon au moins une des revendications 1 à 12, caractérisé en ce qu'on fait agir, sur 100 g à chaque fois du mélange brut de chlorofluoronitrobenzène obtenu dans la seconde étape, 22 g à 81,2 g de chlore ou des quantités équivalentes d'un agent cédant du chlore.

16. Procédé selon au moins une des revendications 1 à 15, caractérisé en ce qu'on fait agir, sur 100 g à chaque fois du mélange brut de chlorofluoronitrobenzène obtenu dans la seconde étape, 22 g à 40,6 g de chlore ou des quantités équivalentes d'un agent cédant du chlore.

17. Procédé selon au moins une des revendications 1 à 16, caractérisé en ce qu'on effectue la chloration dénitrante en présence de pentoxyde de phosphore ou de pentachlorure de phosphore comme agent éliminant l'eau.

18. Procédé selon au moins une des revendications 1 à 17, caractérisé en ce qu'on effectue la chloration dénitrante en présence de chlorure de calcium, d'hydroxyde de calcium, de sulfate de calcium ou de dioxyde de silicium comme agent cédant du fluor.

19. Procédé selon au moins une des revendications 1 à 18, caractérisé en ce qu'on effectue la chloration dénitrante en continu ou en discontinu.
